# FASCICULE DE BREVET EUROPEEN

(11) **EP 1 842 508 B1**
(45) Date de publication et mention de la délivrance du brevet: **17.06.2009**
(21) Numéro de dépôt: 07290371.9
(22) Date de dépôt: 28.03.2007
(51) Int. Cl.: A61F 2/06, A61M 25/00, A61M 29/00

(54) **Dispositif de traitement d'un conduit de circulation du sang et procédé de préparation de ce dispostif**
Vorrichtung zur Behandlung eines Blutschlauchs und Verfahren zur Herstellung dieser Vorrichtung
Device for treating a blood circulation conduit and method of preparing this device

(30) Priorité: 04.04.2006 FR 0602932
(43) Date de publication de la demande: 10.10.2007
(73) Titulaire: Laboratoires Perouse, 60173 Ivry Le Temple (FR)
(72) Inventeur: Styrc, Mikolaj, 8190 Kopstal (LU)
(74) Mandataire: Blot, Philippe Robert Emile

(56) Documents cités:
- EP-A- 0 829 242
- FR-A- 2 865 926
- FR-A- 2 874 812
- US-A- 5 713 948
- US-A- 5 800 506
- US-A1- 2005 119 722

## Description

La présente invention concerne un dispositif de traitement d'un conduit de circulation du sang selon le préambule de la revendication 1.

On connaît de US 2005/0119722 et US-A-5 713 948, un dispositif du type précité.

Un tel dispositif s'applique au largage dans un conduit de circulation du sang d'endoprothèses, du type couramment désignées par le terme anglais « stent », ou d'endoprothèses de type « endovalves » comprenant un stent et une valve fixée sur le stent.

Un dispositif du type précité est décrit dans EP -A- 0 707 462. Dans ce dispositif, une endoprothèse est montée coaxialement sur deux tuteurs creux, aptes à coulisser l'un par rapport à l'autre. Cette endoprothèse est maintenue dans son état rétracté à l'aide de deux liens filiformes la ceinturant à ses extrémités. Les liens filiformes sont passés respectivement dans des ouvertures de retenue distale et proximale ménagées respectivement dans l'un et l'autre des tuteurs. Les liens sont engagés autour d'une tige de retenue pour les maintenir en position à leur extrémité proximale.

Pour le largage de l'endoprothèse, les tuteurs sont déplacés par coulissement relatif l'un par rapport à l'autre, de sorte que la distance entre les ouvertures de retenue diminue.

La diminution de cette distance provoque le relâchement des liens filiformes et par suite, le déploiement simultané des deux extrémités de l'endoprothèse.

Lorsque le placement de l'endoprothèse dans le conduit de circulation du sang est satisfaisant, la tige de retenue des liens filiformes est extraite du dispositif. Les liens filiformes sont alors retirés hors des tuteurs et extraits hors du corps du patient en les faisant glisser dans les tuteurs.

Compte tenu de la grande longueur des liens filiformes, et de leur trajet sinueux dans les tuteurs, le risque de coincement ou de casse des liens filiformes lors de leur retrait est élevé. La fiabilité du dispositif est donc diminuée.

L'invention a donc pour objet de proposer un dispositif de traitement d'un conduit de circulation du sang qui peut être positionné de manière précise dans le vaisseau, tout en présentant une utilisation simple et fiable.

A cet effet, l'invention a pour objet un dispositif de traitement selon la revendication 1..

Le dispositif selon l'invention peut comporter une ou plusieurs des caractéristiques des revendications 2 à 11..

L'invention a également pour objet un procédé de préparation que décrit ci-dessus, avant son implantation dans un conduit de circulation du sang selon la revendication 12.

L'invention sera mieux comprise à la lecture de la description qui va suivre, donnée uniquement à titre d'exemple, et faite en se référant aux dessins annexés sur lesquels :
- la Figure 1 est une vue en élévation d'un premier dispositif de traitement selon l'invention, l'endoprothèse étant maintenue dans son état rétracté ;
- la Figure 2 est une vue en coupe, prise suivant le plan transversal médian II-II, d'un détail de la Figure 1 ;
- la Figure 3 est une vue analogue à la Figure 1, l'endoprothèse présentant son état dilaté ;
- la Figure 4 est une vue analogue à la Figure 2 de l'endoprothèse de la Figure 3;
- la Figure 5 est une vue en perspective de trois-quarts face de l'endoprothèse du dispositif de la Figure 1 dans son état dilaté ;
- la Figure 6 est une vue analogue à la Figure 2 d'un détail d'un deuxième dispositif de traitement selon l'invention ;
- la Figure 7 est une vue analogue à la Figure 6 d'un troisième dispositif selon l'invention ;
- la Figure 8 est une vue analogue à la Figure 6 d'un quatrième dispositif selon l'invention, l'endoprothèse n'étant pas représentée ; et
- la Figure 9 est une vue analogue à la Figure 5 de l'endoprothèse du dispositif de la Figure 8.

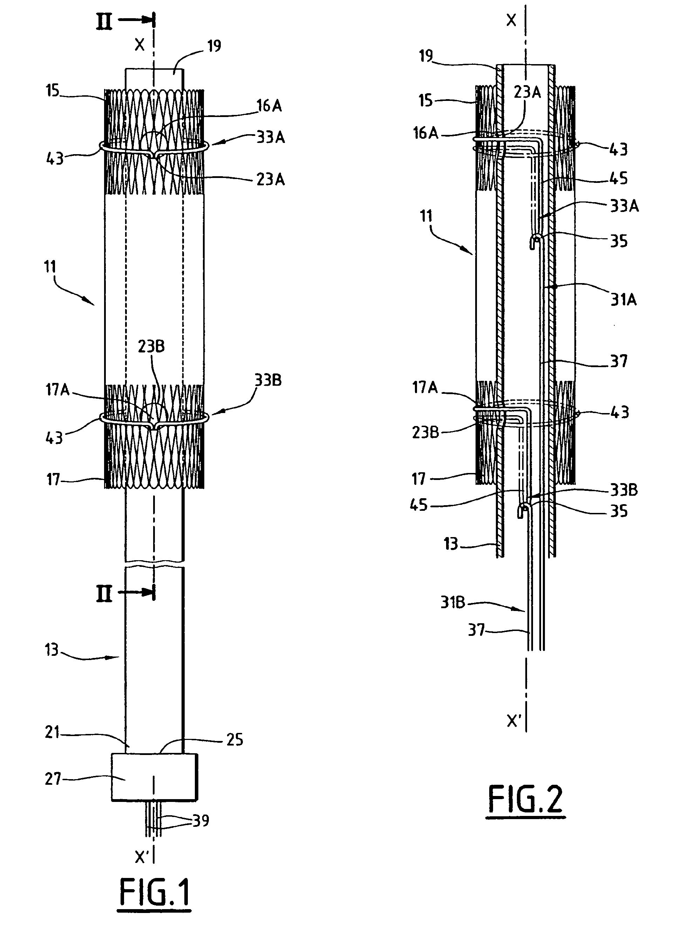

Le dispositif de traitement représenté sur les Figures 1 à 5 comporte une endoprothèse tubulaire 11 d'axe X-X', montée de manière coaxiale sur un tuteur unique 13 et reliée à ce tuteur 13 par des moyens de retenue libérables.

L'endoprothèse 11 comprend un treillis tubulaire d'acier inoxydable qui possède des propriétés de ressort. Ainsi, cette endoprothèse est auto-expansible.

Cette endoprothèse 11 est par exemple réalisée par tressage d'un fil unique d'un matériau superélastique, comme décrit dans la demande de brevet européen EP-A-0 857 471.

Le treillis de l'endoprothèse 11 définit, au voisinage d'une extrémité distale 15 de l'endoprothèse 11, un passage 16A de guidage distal des moyens de retenue, et au voisinage d'une extrémité proximale 17 de l'endoprothèse 11, un passage de guidage proximal 17A des moyens de retenue.

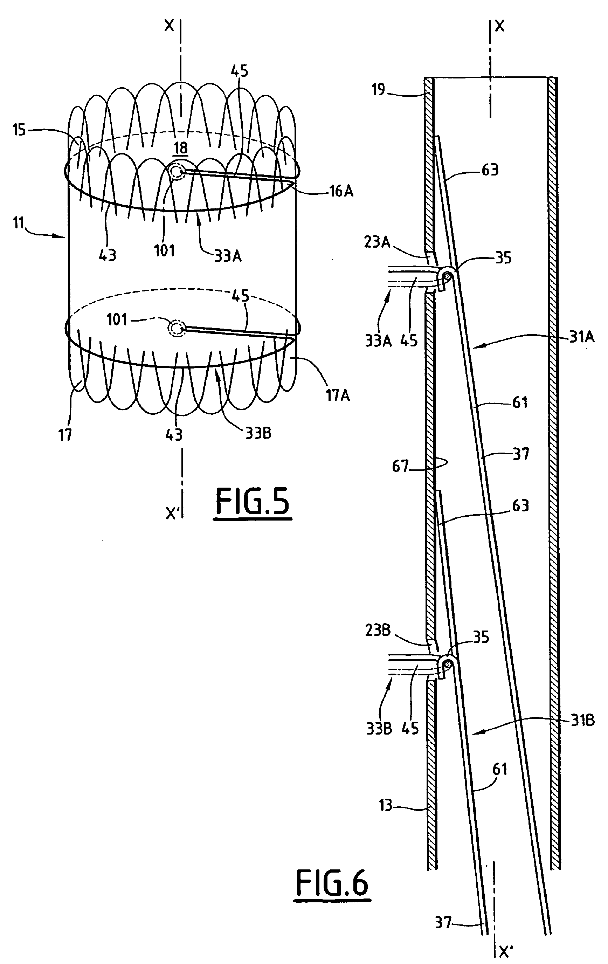

Comme illustré par la Figure 5, chaque passage de guidage 16A, 17A est délimité par une maille du treillis de l'endoprothèse. Les passages 16A, 17A sont situés sur la même génératrice longitudinale de l'endoprothèse 11.

Le treillis présente aux extrémités 15, 17 de l'endoprothèse 11 des fils repliés formant des coudes.

L'endoprothèse 11 délimite intérieurement un conduit 18 de circulation du sang d'axe X-X'.

En variante, les passages 16A, 17A sont délimités par des anneaux solidaires du treillis et disposés dans le conduit 18.

Comme connu en soi, l'endoprothèse 11 est susceptible de se déformer spontanément d'un état rétracté, dans lequel elle présente un petit diamètre (Figure 1) à un état dilaté, dans lequel elle présente un diamètre supérieur (Figure 5), cet état dilaté constituant son état de repos.

Dans l'exemple illustré sur les Figures 1 et 2, le tuteur 13 comprend un tube métallique flexible creux d'axe X-X'. Le diamètre interne du tube est adapté pour enfiler celui-ci sur un guide chirurgical filiforme (non représenté) installé sur le patient, préalablement à la mise en place de l'endoprothèse 11 dans un conduit de circulation du sang de ce patient.

Le tuteur 13 s'étend longitudinalement d'un seul tenant entre une extrémité distale 19 destinée à être implantée dans le conduit de circulation du sang et une extrémité proximale 21 destinée à être accessible pour un chirurgien. L'extrémité proximale 21 est située à l'écart de l'endoprothèse 11.

Des ouvertures de retenue distale et proximale 23A et 23B, décalées longitudinalement, sont ménagées latéralement dans le tuteur 13, au voisinage de l'extrémité distale 19 du tuteur 13.

Dans cet exemple, les ouvertures 23A et 23B sont ménagées du même coté par rapport à un plan longitudinal médian du tuteur 13. La distance qui sépare l'ouverture de retenue distale 23A de l'ouverture de retenue proximale 23B est sensiblement égale à la longueur de l'endoprothèse 11 dans son état rétracté, prise suivant une direction longitudinale.

Le tuteur 13 délimite à son extrémité proximale 21 un passage de commande 25 obturé de manière sélective par des moyens 27 de blocage en position des moyens de retenue.

Les moyens de retenue de l'endoprothèse 11 comprennent une tige de retenue distale 31A et une tige de retenue proximale 31 B, associées respectivement à un fil de retenue distal 33A et à un fil de retenue proximal 33B.

La tige de retenue distale 31A et la tige de retenue proximale 31B sont de structures analogues. De même, le fil de retenue distal 33A et le fil de retenue proximal 33B sont de structures analogues. Par suite, seuls le fil de retenue distal 33A et la tige de retenue distale 31A seront décrits ci-dessous.

La tige de retenue 31A est disposée dans le tuteur 13. La longueur de la tige 31A est supérieure ou égale à la distance entre l'ouverture de retenue distale 23A et l'extrémité proximale 21 du tuteur 13.

Comme illustré sur la Figure 2, la tige 31A comprend un crochet d'extrémité 35, une partie d'actionnement 37, et un tronçon de commande 39 qui fait saillie à travers le passage de commande 25 à l'extrémité proximale 21 du tuteur 13. La tige de retenue 31A présente ainsi une forme générale de crosse.

Le crochet d'extrémité 35 est situé à l'extrémité distale de la partie d'actionnement 37. Il fait saillie latéralement par rapport à la partie d'actionnement 37 vers l'ouverture de retenue distale 23A.

La partie d'actionnement 37 est réalisée à base d'une tige métallique flexible. Elle s'étend dans le tuteur 13.

Le tronçon de commande 39 prolonge la partie d'actionnement 37 à l'extérieur du tuteur 13, à travers le passage de commande 25.

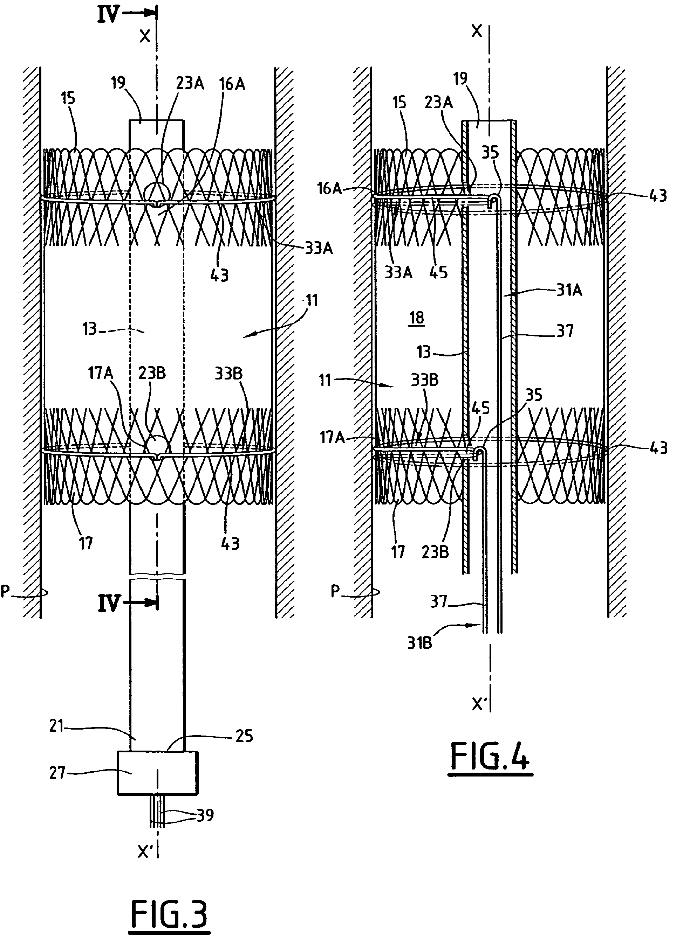

La tige 31A est mobile en translation le long de l'axe X-X' du tuteur 13, entre une position distale de libération du fil de retenue 33A représentée sur la Figure 4, et une position proximale de traction du fil de retenue 33A représentée sur la Figure 2.

Dans la position distale de libération, le crochet 35 s'étend sensiblement en regard de l'ouverture de retenue distale 23A. Dans cette position, le tronçon 39 de commande présente une longueur minimale.

Dans la position proximale de traction, le crochet 35 est situé entre l'ouverture de retenue proximale 23B et l'ouverture de retenue distale 23A. La longueur du tronçon de commande 39 est alors maximale.

Dans l'exemple représenté sur les Figures 1 à 5, le fil de retenue distal 33A comprend un brin unique de matériau souple fermé sur lui-même. Le fil de retenue distal 33A forme ainsi une boucle de serrage 43, située à l'extérieur du tuteur 13 pour ceinturer l'endoprothèse 11, et un passant 45 engagé dans le tuteur 13 à travers l'ouverture de retenue distale 23A.

Le fil de retenue distal 33A est monté à demeure sur l'endoprothèse 11.

La boucle de serrage 43 ceinture l'endoprothèse 11 à l'extérieur du treillis, suivant une circonférence autour de l'axe X-X'. Elle s'étend entre une première extrémité et une deuxième extrémité engagées dans le passage de guidage proximal 16A et raccordées au passant 45.

En variante, la boucle de serrage 43 est engagée dans le treillis de l'endoprothèse 11, suivant une circonférence, en passant successivement à l'intérieur et à l'extérieur du treillis.

Le passant 45 s'étend dans le tuteur 13 en raccordant les deux extrémités de la boucle de serrage 43 à travers l'ouverture de retenue 23A. Le passant 45 est engagé dans le crochet 35 de la tige de retenue 31A.

La boucle de serrage 43 est extensible entre une configuration de maintien de l'endoprothèse 11 dans son état rétracté, et une configuration de déploiement de l'endoprothèse 11.

Dans la configuration de maintien représentée sur la Figure 2, le diamètre de la boucle de serrage 43 est minimal, alors que la longueur du passant 45 dans le tuteur 13 est maximale.

A l'inverse, comme représenté sur la Figure 4, lorsque la boucle de serrage 43 occupe sa configuration de déploiement, son diamètre est maximale, alors que la longueur du passant 45 dans le tuteur 13 est minimale.

Lorsque la boucle de serrage 43 occupe sa configuration de maintien de l'endoprothèse 11, l'endoprothèse 11 est maintenue dans son état rétracté contre le tuteur 13, le long d'une surface latérale périphérique du tuteur 13. A l'inverse, lorsque la boucle de serrage 43 occupe sa configuration de déploiement, l'endoprothèse 11 est libre d'occuper son état dilaté.

On décrira maintenant comme exemple le fonctionnement du premier dispositif de traitement selon l'invention.

Dans un premier temps, le dispositif est conservé dans un emballage stérile (non représenté).

Dans l'emballage, chaque tige de retenue 31 occupe sensiblement sa position de libération du passant 45, dans laquelle le crochet 35 est situé en regard d'une ouverture de retenue 23A, 23B.

Pour chaque fil 33A, 33B, le passant 45 reste engagé dans un crochet 35 associé, de sorte que la tige de retenue 31A, 31 B retient le fil de retenue 33A, 33B.

Les fils de retenue distal et proximal 33A et 33B sont engagés autour du treillis de l'endoprothèse 11, de sorte que chaque boucle de serrage 43 occupe sa configuration de déploiement. L'endoprothèse 11 est ainsi maintenue dans son état dilaté.

Ce conditionnement conserve les propriétés mécaniques de l'endoprothèse 11, particulièrement le treillis tubulaire de celle-ci est noyé dans un film extensible et étanche, tel qu'un élastomère.

Dans un deuxième temps, le chirurgien implante un guide chirurgical (non représenté) circulant dans le conduit de circulation du sang ou la veine depuis le point d'introduction extérieur jusqu'à la zone de la veine ou de l'artère dans laquelle doit être implantée l'endoprothèse tubulaire 11.

Puis, le chirurgien extrait le dispositif de son emballage en vue de l'implantation de l'endoprothèse 11 dans le conduit de circulation du sang ou la veine. Le chirurgien libère alors les moyens de blocage 27 des tiges 31A, 31B et tire sur les tronçons de commande 39 des tiges de retenue 31A, 31 B, afin d'augmenter la longueur de ces tronçons 39.

Lors de ce déplacement, chaque crochet 35 se déplace vers l'extrémité proximale 21 du tuteur 13, jusqu'à ce que les tiges de retenue 31A, 31B occupent leur position de retenue du passant.

Lors de ce déplacement, chaque tige de retenue 31A, 31B tire sur un fil de retenue 33A, 33B correspondant. La longueur de chaque passant 45 augmente, et le diamètre des boucles de serrage 43 diminue de manière correspondante. L'endoprothèse 11 passe ainsi de son état dilaté à son état rétracté contre une surface latérale périphérique du tuteur 13, dans lequel le treillis est sensiblement en appui contre le tuteur 13, autour de ce tuteur 13. Le tuteur 13 est alors situé dans le conduit de circulation 18.

Le chirurgien active alors les moyens 27 de blocage en position des tiges de retenue 31A, 31B pour immobiliser les tiges 31A, 31 B par rapport au tuteur 13.

L'endoprothèse 11 est ainsi introduite coaxialement avec le tuteur 13 jusqu'à son lieu d'implantation par déplacement le long du guide chirurgical (non représenté).

Dans certains cas, et pour maintenir un encombrement radial minimal, un fourreau (non représenté) est disposé autour de l'endoprothèse 11, avant cette introduction, et retiré une fois l'introduction effectuée.

Une fois l'endoprothèse 11 introduite, le chirurgien procède à son déploiement.

En fonction de la conformation du conduit de circulation du sang à traiter, il peut choisir de déployer en premier l'une ou l'autre des extrémités 15 et 17 de l'endoprothèse 11.

On décrira comme exemple le déploiement de l'extrémité distale 15.

Tout d'abord, le chirurgien libère sélectivement le tronçon de commande 39 de la tige de retenue distale 31A. Puis, il diminue progressivement la longueur de ce tronçon 39 en déplaçant la tige 31A vers l'extrémité distale 19 du tuteur 13, ce qui déplace le crochet 35 vers l'extrémité distale 19.

Lors de ce déplacement, la longueur du passant 45 diminue. Le diamètre de la boucle de serrage 43 augmente de manière correspondante, ce qui permet le déploiement de l'endoprothèse 11 radialement par rapport à l'axe X-X' du tuteur 13 au niveau de l'extrémité distale 15 de l'endoprothèse 11. Le treillis s'éloigne donc du tuteur 13 et se rapproche des parois P du conduit à traiter pour venir en appui sur ces parois P.

Si le chirurgien n'est pas satisfait du positionnement de l'extrémité distale 15 de l'endoprothèse 11 lorsque celle-ci est déployée, il actionne de nouveau le tronçon de commande 39 en déplaçant la tige de retenue 31A vers l'extrémité proximale 21 du tuteur 13. Ceci augmente la longueur du passant 45 et diminue la longueur active de la boucle de serrage 43 afin de comprimer l'endoprothèse 11 contre le tuteur 13. L'endoprothèse 11 est alors déplacée jusqu'à une position plus satisfaisante.

De manière analogue, le chirurgien effectue ensuite le déploiement de l'extrémité proximale 17 de l'endoprothèse 11 au moyen du fil de retenue proximal 33B.

Dans cette configuration, les tiges de retenue 31A, 31B occupent leur position de libération du passant 45. Les passants 45 restent toutefois engagés dans les crochets 35.

Lorsque le chirurgien est satisfait du positionnement de l'extrémité distale 15 de l'endoprothèse 11, il déplace la tige de retenue distale 31A vers l'extrémité distale 19 du tuteur 13 pour amener le crochet 35 au-delà de l'ouverture de retenue distale 23A, jusqu'à ce que le passant 45 se libère totalement du crochet 35.

L'extrémité distale 15 de l'endoprothèse 11 est alors fixée de manière irréversible sur les parois P du conduit de circulation du sang.

Le chirurgien procède alors de même pour l'extrémité proximale 17 de l'endoprothèse 11. Puis, il extrait le tuteur 13 et les tiges de retenue 31A, 31B hors du patient. Le tuteur 13 est alors totalement libéré de l'endoprothèse 11.

Dans ce dispositif, la longueur des fils de retenue 31A, 31B est minimale, de sorte que leur circulation dans le tuteur 13 présente de faibles risques de blocage.

Par suite, la fiabilité du déploiement de l'endoprothèse est améliorée, puisqu'il n'est plus nécessaire de faire circuler de grandes longueurs de fil dans le tuteur 13.

Dans une variante représentée en pointillés sur la Figure 5, chaque passant 45 présente un anneau 101 d'extrémité, par exemple réalisé en un matériau radio-opaque, destiné à être saisi par un crochet 35.

Dans le deuxième dispositif selon l'invention, représenté sur la Figure 6, le crochet 35 de chaque tige de retenue 31A, 31B n'est pas situé à l'extrémité distale de la tige 31A, 31B.

Ainsi, chaque crochet 35 délimite sur la tige 31A, 31 B un tronçon proximal 61 et un tronçon distal 63 de longueur non nulle, apte à retenir le crochet 35 dans le tuteur 13. Le crochet 35 fait saillie radialement par rapport au tronçon proximal 61 et au tronçon distal 63.

Lorsque la tige de retenue 31A, 31B est placée dans sa position de libération du passant 45, avec le crochet 35 situé en regard d'une ouverture de retenue 23A, 23B, le tronçon distal 63 prend appui contre la surface intérieure 67 du tuteur 13, distalement par rapport à l'ouverture 23A, 23B. Ceci évite que le crochet 35 ne soit entraîné par le passant 45 hors du tuteur 13 à travers l'ouverture de retenue 23A, 23B lors du déploiement de l'endoprothèse 11. La fiabilité du dispositif est encore améliorée.

En variante (non représentée), les deux tiges de retenue 31A, 31B sont raccordées mécaniquement en un point situé proximalement par rapport à l'ouverture de retenue proximale 23B lorsque les crochets 35 sont situés en regard des ouvertures de retenue 23A, 23B.

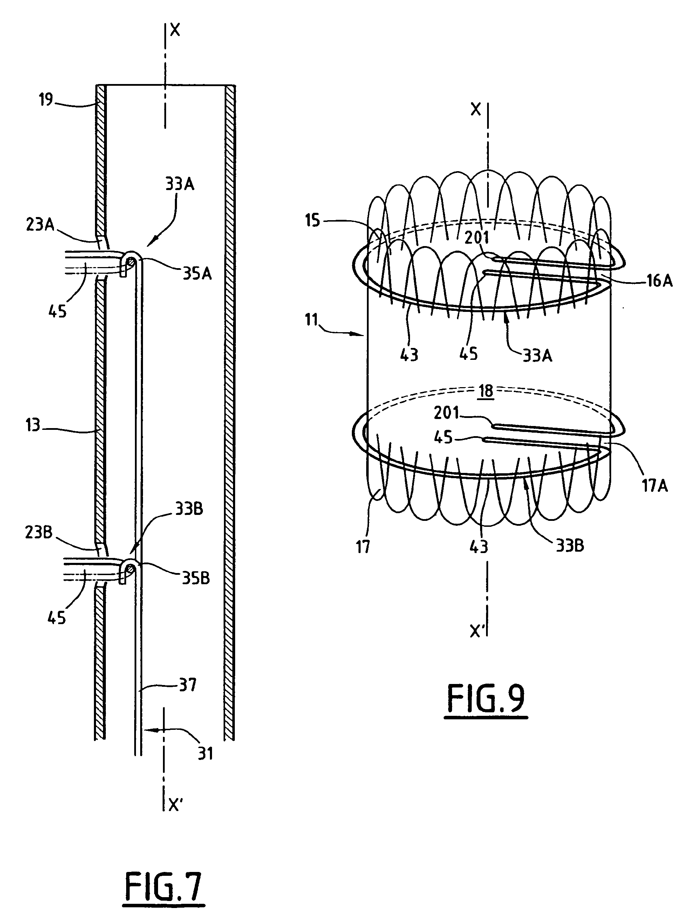

Le troisième dispositif selon l'invention, représenté sur la Figure 7, comprend une tige de retenue unique 31 munie d'un crochet de retenue distal 35A situé à l'extrémité distale de la tige 31, et d'un crochet de retenue proximal 35B qui fait saillie par rapport à la tige 31.

La distance qui sépare le crochet distal 35A du crochet proximal 35B est sensiblement égale à la distance qui sépare l'ouverture de retenue distale 23A de l'ouverture de retenue proximale 23B.

Ce troisième dispositif permet le déploiement simultané de l'extrémité proximale 17 et de l'extrémité distale 15 de l'endoprothèse 11.

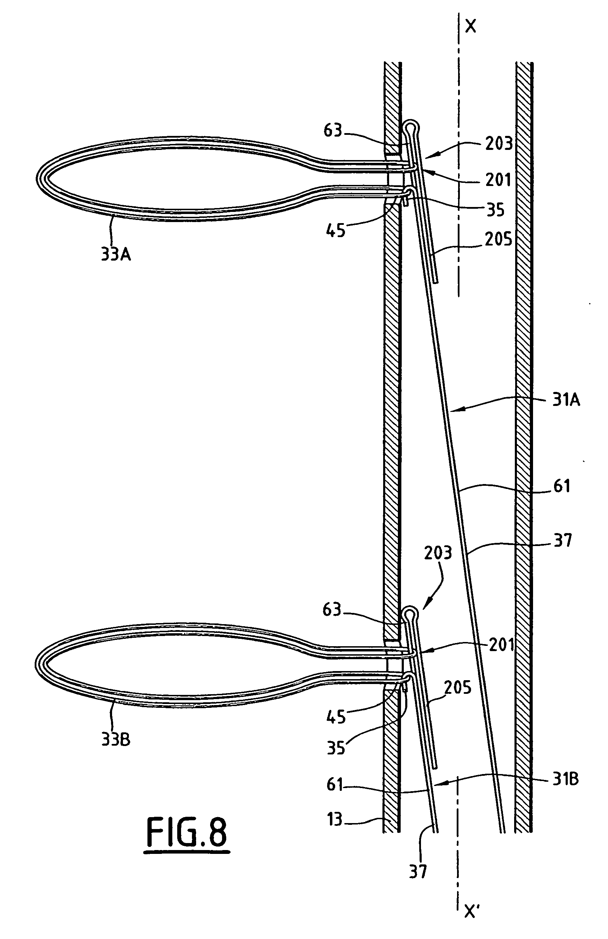

Un quatrième dispositif selon l'invention est représenté sur les Figures 8 et 9. Ce dispositif constitue une variante du deuxième dispositif de traitement selon l'invention. Toutefois, à la différence du deuxième dispositif, chaque fil de retenue 33A, 33B est libérable de l'endoprothèse 11.

A cet effet, chaque fil de retenue 33A, 33B forme un passant principal 45 raccordé à une première extrémité de la boucle de serrage 43, et un passant auxiliaire 201 disposé dans le tuteur 13 et raccordé à une deuxième extrémité de la boucle de serrage 43.

La boucle de serrage 43 est formée par deux brins adjacents du fil de retenue 33A, 33B. Ces deux brins sont repliés en boucle au niveau de la première extrémité de la boucle de serrage 43 pour former le passant principal 45 et sont repliés en boucle au niveau de la deuxième extrémité de la boucle de serrage 43 pour former le passant auxiliaire 201.

Le passant principal 45 et le passant auxiliaire 201 sont raccordés à la boucle de serrage 43 à travers la même ouverture de retenue 23A, 23B et à travers le même passage de guidage 16A, 17A.

A la différence du dispositif représenté sur la Figure 6, chaque tige de retenue 31A, 31B présente à son extrémité distale une pince de retenue 203 du passant auxiliaire 201.

La pince 203 est formée par un repli 205 du tronçon distal 63 de la tige de retenue 31A, 31B s'étendant entre l'extrémité distale de la tige 31A, 31B jusqu'à un point situé de manière proximale par rapport au crochet de retenue 35. Le repli 205 s'étend à l'opposé du crochet 35 et de l'ouverture de retenue 23A, 23B, par rapport à l'axe de la tige 31A, 31 B. La pince 203 s'ouvre vers l'extrémité proximale 21 du tuteur 13.

Le passant auxiliaire 201 est engagé dans la pince de retenue 203.

Lorsque le chirurgien extrait le quatrième dispositif de son emballage et qu'il déplace chaque tige de retenue 31A, 31B vers l'extrémité proximale 21 du tuteur 13. Le crochet 35 et la pince 203 de chaque tige 31A, 31B se déplacent également vers cette extrémité proximale.

Lors de ce déplacement, le passant 45 et le passant auxiliaire 201 sont tirés dans le tuteur 13 vers l'extrémité proximale 21 de sorte que la longueur de la boucle de serrage 43 diminue pour faire passer l'endoprothèse 11 de son état dilaté à son état rétracté contre le tuteur 13.

Puis, le déploiement de l'endoprothèse 11 est analogue à celui du deuxième dispositif de traitement.

Lorsque l'endoprothèse 11 est déployée en position dans le conduit de circulation du sang, et que le chirurgien est satisfait du positionnement de cette endoprothèse, il déplace la tige de retenue 31A, 31B vers l'extrémité distale 19 du tuteur 13 pour amener le crochet 35 au-delà de l'ouverture de retenue correspondante 23A, 23B. Le passant principal 45 se libère alors totalement du crochet 35.

Toutefois, compte tenu de la longueur du repli 205, le passant auxiliaire 201 reste engagé dans la pince 203.

Ensuite, lorsque le chirurgien tire la tige de retenue 31A, 31B vers l'extrémité proximale 21 du tuteur 13, il tire également sur le fil de retenue 33A, 33B correspondant par l'intermédiaire du passant auxiliaire 201 engagé dans la pince 203 pour permettre un déplacement conjoint du fil de retenue 33A, 33B et de la tige de retenue 31A, 31B. Le fil de retenue 33A, 33B est alors libéré totalement de l'endoprothèse 11, et extrait hors du patient en même temps que la tige de retenue 31A, 31B.

## Revendications

1. Dispositif de traitement d'un conduit de circulation du sang, du type comprenant :
- un tuteur creux (13) **unique** délimitant, au voisinage d'une extrémité distale (19), au moins une ouverture transversale (23A, 23B) de retenue ; **le tuteur unique présentant des ouvertures de retenue distale et proximale, décalées longitudinalement, ménagées latéralement dans le tuteur ;**
- une endoprothèse (11) déployable entre un état rétracté contre une surface latérale du tuteur (13) et un état dilaté dans lequel l'endoprothèse (11) peut être libérée du tuteur (13) ;
- au moins **un lien filiforme de retenue distal (33A) et un lien filiforme de retenue proximal (33B), chaque** lien filiforme (33A, 33B) formant une boucle de serrage (43) ceinturant l'endoprothèse (11) et formant un passant (45) engagé dans le tuteur **unique** (13) à travers l'ouverture de retenue (23A, 23B) ;
la boucle de serrage (43) étant extensible entre une configuration de maintien de l'endoprothèse (11) dans son état rétracté et une configuration de déploiement de l'endoprothèse (11) ; et pour chaque lien filiforme (33A, 33B),
- une tige de retenue (31A, 31 B) montée mobile dans le tuteur (13) entre une position de retenue du passant (45) et une position de libération du passant (45) ;
**caractérisé en ce que pour chaque lien filiforme,** la tige (31A, 31B) de retenue comprend au moins un crochet (35) de retenue du passant (45), le déplacement de la tige de retenue (31A, 31 B) depuis sa position de libération vers sa position de retenue provoquant **l'augmentation de la longueur du passant, la diminution correspondante de la longueur de la boucle de serrage et** le serrage de la boucle de serrage (43) depuis sa configuration de déploiement de l'endoprothèse (11) vers sa configuration de maintien de l'endoprothèse (11).

2. Dispositif selon la revendication 1, **caractérisé en ce que** le crochet (35) est placé à une extrémité distale de la tige de retenue (31A ; 31B).

3. Dispositif selon la revendication 1, **caractérisé en ce que** le crochet (35) délimite, sur la tige de retenue (31A, 31 B), un tronçon distal (63) de longueur non nulle et un tronçon proximal (61), le crochet (35) faisant saillie transversalement par rapport au tronçon distal (63) et au tronçon proximal (61).

4. Dispositif selon l'une quelconque des revendications précédentes, **caractérisé en ce que** l'endoprothèse (11) délimite, pour le ou pour chaque lien filiforme (33A, 33B), un passage (16A, 17A) de guidage du lien filiforme (33A, 33B), les extrémités de la boucle de serrage (43) étant engagées dans le passage de guidage (16A, 17A).

5. Dispositif selon la revendication 4, **caractérisé en ce que** le ou chaque lien filiforme (33A, 33B) est un brin fermé sur lui-même, le passant (45) étant formé par un prolongement de la boucle de serrage (43) s'étendant à partir du passage de guidage (16A, 17A) entre les extrémités de la boucle de serrage (43).

6. Dispositif selon l'une quelconque des revendications précédentes, **caractérisé en ce que** le ou chaque lien filiforme (31A, 31B) est monté à demeure sur l'endoprothèse (11).

7. Dispositif selon l'une quelconque des revendications précédentes, **caractérisé en ce que** le tuteur (13) délimite une ouverture de retenue proximale (23B) et une ouverture de retenue distale (23A), le dispositif comprenant, pour chaque ouverture de retenue (23A, 23B), une tige de retenue distincte (31A, 31 B) comprenant un crochet de retenue (35).

8. Dispositif selon l'une quelconque des revendications 1 à 6, **caractérisé en ce que** le tuteur (13) délimite une ouverture de retenue proximale (23B) et une ouverture de retenue distale (23A), le dispositif comprenant une tige de retenue commune (31) comprenant un crochet distal (35A) et un crochet proximal (35B).

9. Dispositif selon l'une quelconque des revendications précédentes, **caractérisé en ce que** l'endoprothèse (11) est montée coaxialement sur le tuteur (13).

10. Dispositif selon l'une quelconque des revendications précédentes, **caractérisé en ce que** le ou chaque lien filiforme (33A, 33B) est libérable de l'endoprothèse (11), la tige de retenue (31A, 31 B) comprenant des moyens (203) de retenue d'au moins un tronçon de retenue (201) du lien filiforme (33A, 33B) distinct du passant (45), le tronçon (201) étant retenu par les moyens de retenue (203) lorsque le passant (45) est libéré du crochet de retenue (35), pour permettre le déplacement conjoint du lien filiforme (33A, 33B) et de la tige de retenue (31A, 31B).

11. Dispositif selon la revendication 10, **caractérisé en ce que** le tronçon de retenue (201) du lien filiforme (33A, 33B) forme un passant auxiliaire engagé dans le tuteur (13) à travers une ouverture de retenue (23A, 23B), la tige de retenue comprenant un crochet auxiliaire (203) de retenue du passant auxiliaire (201) s'ouvrant de manière proximale par rapport au crochet de retenue (35) du passant (45).

12. Procédé de préparation d'un dispositif selon l'une quelconque des revendications précédentes, avant son implantation dans un conduit de circulation du sang, **caractérisé en ce qu'**il comprend les étapes suivantes :
(a) conservation de l'endoprothèse (11) dans son état dilaté, le ou chaque passant (45) étant engagé dans un crochet (35) d'une tige de retenue (31A, 31 B) du passant (45) ;
(b) déplacement de la tige de retenue (31A, 31 B) depuis sa position de libération vers la position de retenue du passant (45), pour provoquer le serrage de la boucle de serrage (43) et pour amener l'endoprothèse (11) dans son état rétracté.

## Claims

1. Device for treating a blood circulation conduit, of the type comprising:
- a single hollow stent (13) delimiting, close to a distal end (19), at least one transverse retaining opening (23A, 23B); the single stent having distal and proximal retaining openings which are longitudinally offset and formed laterally in the stent;
- an endoprosthesis (11) which can be deployed between a state in which it is retracted against a lateral surface of the stent (13) and an expanded state in which the endoprosthesis (11) may be freed from the stent (13);
- at least one filiform distal retaining fastener (33A) and a filiform proximal retaining fastener (33B), each filiform fastener (33A, 33B) forming a tightening loop (43) encircling the endoprosthesis (11) and forming a eyelet (45) engaging in the single stent (13) through the retaining opening (23A, 23B);
- the tightening loop (43) being extensible between a configuration for holding the endoprosthesis (11) in its retracted state and a configuration of deployment of the endoprosthesis (11); and for each filiform fastener (33A, 33B),
- a retaining rod (31A, 31B) mounted so as to be movable in the stent (13) between a retaining position of the eyelet (45) and a position of release of the eyelet (45);
**characterised in that** for each filiform fastener, the retaining rod (31A, 31B) comprises at least one retaining hook (35) for the eyelet (45), the movement of the retaining rod (31 A, 31 B) from its release position to its retaining position causing an increase in the length of the eyelet, the corresponding reduction in the length of the tightening loop and the tightening of the tightening loop (43) from its configuration of deployment of the endoprosthesis (11) into its configuration of holding the endoprosthesis (11).

2. Device according to claim 1, **characterised in that** the hook (35) is placed at a distal end of the retaining rod (31A; 31B).

3. Device according to claim 1, **characterised in that** the hook (35) delimits, on the retaining rod (31A, 31B), a distal section (63) with a length other than zero and a proximal section (61), the hook (35) projecting transversely relative to the distal section (63) and the proximal section (61).

4. Device according to any one of the preceding claims, **characterised in that** the endoprosthesis (11) delimits, for the or each filiform fastener (33A, 33B), a guide passage (16A, 17A) for the filiform fastener (33A, 33B), the ends of the tightening loop (43) engaging in the guide passage (16A, 17A).

5. Device according to claim 4, **characterised in that** the or each filiform fastener (33A, 33B) is a strand closed in on itself, the eyelet (45) being formed by an extension of the tightening loop (43) extending from the guide passage (16A, 17A) between the ends of the tightening loop (43).

6. Device according to any one of the preceding claims, **characterised in that** the or each filiform fastener (33A, 33B) is permanently mounted on the endoprosthesis (11).

7. Device according to any one of the preceding claims, **characterised in that** the stent (13) delimits a proximal retaining opening (23B) and a distal retaining opening (23A), the device comprising, for each retaining opening (23A, 23B), a distinct retaining rod (31 A, 31B) comprising a retaining hook (35).

8. Device according to any one of claims I to 6, **characterised in that** the stent (13) delimits a proximal retaining opening (23B) and a distal retaining opening (23A), the device comprising a common retaining rod (31) comprising a distal hook (35A) and a proximal hook (35B).

9. Device according to any one of the preceding claims, **characterised in that** the endoprosthesis (11) is mounted coaxially on the stent (13).

10. Device according to any one of the preceding claims, **characterised in that** the or each filiform fastener (33A, 33B) can be freed from the endoprosthesis (11), the retaining rod (31A, 31B) comprising means (203) for retaining at least one retaining section (201) of the filiform fastener (33A, 33B) distinct from the eyelet (45), the section (201) being held by the retaining means (203) when the eyelet (45) is released from the retaining hook (35), to allow joint movement of the filiform fastener (33A, 33B) and the retaining rod (31A, 31B).

11. Device according to claim 10, **characterised in that** the retaining section (201) of the filiform fastener (33A, 33B) forms an auxiliary eyelet engaging in the stent (13) through a retaining opening (23A, 23B), the retaining rod comprising an auxiliary hook (203) for retaining the auxiliary eyelet (201), which opens proximally relative to the retaining hook (35) of the eyelet (45).

12. Process for preparing a device according to any one of the preceding claims, before it is implanted in a blood circulation conduit, **characterised in that** it comprises the following steps:
(a) maintaining the endoprosthesis (11) in its expanded state, the or each eyelet (45) engaging in a hook (35) of a retaining rod (31A, 31B) of the eyelet (45);
(b) moving the retaining rod (31A, 31B) from its position of release to the position of retaining the eyelet (45), so as to cause the tightening loop (43) to be tightened and to bring the endoprosthesis (11) into its retracted state.

## Patentansprüche

1. Vorrichtung zur Behandlung eines Blutkreislaufkanals des Typs, umfassend:
- einen einzelnen hohlen Stent (13), der in der Nähe eines distalen Ende (19) wenigstens eine transversale Halteöffnung (23A, 23B) begrenzt; wobei der einzige Stent distale und proximale Halteöffnungen longitudinal in dem Stent versetzt aufweist, die lateral in dem Stent angeordnet sind;
- eine Endoprothese (11), die zwischen einem gegen eine Seitenfläche des Stents (13) eingeschobenen und einem erweiterten Zustand entfaltet werden kann, in dem die Endoprothese (11) von dem Stent (13) gelöst werden kann;
- wenigstens ein distales fadenförmiges Halteband (33A) und ein proximales fadenförmiges Halteband (33B), wobei jedes fadenförmige Halteband (33A, 33B) eine Halteschleife (43) bildet, die die Endoprothese (11) umgibt und eine Schlaufe (45) bildet, die in dem einzigen Stent (13) durch die Halteöffnung (23A, 23B) eingehakt wird;
wobei die Halteschleife (43) zwischen einer Konfiguration des Haltens der Endoprothese (11) in ihrem eingeschobenen Zustand und einer Konfiguration des Entfaltens der Endoprothese (11) erweiterbar ist; und für jedes fadenförmige Halteband (33A, 33B)
- einen Haltestab (31A, 31B), der in dem Stent (13) zwischen einer Halteposition der Schlaufe (45) und einer Löseposition der Schlaufe (45) beweglich montiert ist;
**dadurch gekennzeichnet, dass** der Haltestab (31A, 31B) für jedes fadenförmige Halteband wenigstens einen Haken (35) zum Halten der Schlaufe (45) umfasst, wobei eine Bewegung des Haltestabs (31A, 31B) von seiner Löseposition zu seiner Halteposition eine Verlängerung der Schlaufe, eine entsprechende Verkürzung der Halteschleife und ein Halten der Halteschleife (43) von ihrer Konfiguration des Entfaltens der Endoprothese (11) zu ihrer Konfiguration des Haltens der Endoprothese (11) bewirkt.

2. Vorrichtung nach Anspruch 1, **dadurch gekennzeichnet, dass** der Haken (35) an einem distalen Ende des Haltestabs (31A, 31B) platziert ist.

3. Vorrichtung nach Anspruch 1, **dadurch gekennzeichnet, dass** der Haken (35) am Haltestab (31A, 31B) einen distalen Abschnitt (63) mit einer Länge von ungleich null und einen proximalen Abschnitt (61) begrenzt, wobei der Haken (35) transversal in Bezug auf den distalen Abschnitt (63) und den proximalen Abschnitt (61) herausragt.

4. Vorrichtung nach einem der vorherigen Ansprüche, **dadurch gekennzeichnet, dass** die Endoprothese (11) für das oder jedes fadenförmige Halteband (33A, 33B) eine Führungspassage (16A, 17A) des fadenförmigen Haltebandes (33A, 33B) begrenzt, wobei die Enden der Halteschleife (43) in die Führungspassage (16A, 17A) eingehakt werden.

5. Vorrichtung nach Anspruch 4, **dadurch gekennzeichnet, dass** das oder jedes fadenförmige Halteband (33A, 33B) ein in sich geschlossener Faden ist, wobei die Schlaufe (45) durch eine Verlängerung der Halteschleife (43) gebildet wird, die von der Führungspassage (16A, 17A) zwischen den Enden der Halteschleife (43) ausgehend verläuft.

6. Vorrichtung nach einem der vorherigen Ansprüche, **dadurch gekennzeichnet, dass** das oder jedes fadenförmige Halteband (31A, 31B) an der Endoprothese (11) stationär montiert ist.

7. Vorrichtung nach einem der vorherigen Ansprüche, **dadurch gekennzeichnet, dass** der Stent (13) eine proximale Halteöffnung (23B) und eine distale Halteöffnung (23A) begrenzt, wobei die Vorrichtung für jede Halteöffnung (23A, 23B) einen separaten Haltestab (31A, 31B) umfasst, der einen Haltehaken (35) umfasst.

8. Vorrichtung nach einem der Ansprüche 1 bis 6, **dadurch gekennzeichnet, dass** der Stent (13) eine proximale Halteöffnung (23B) und eine distale Halteöffnung (23A) begrenzt, wobei die Vorrichtung einen gemeinsamen Haltestab (31A, 31B) umfasst, der einen distalen Haken (35A) und einen proximalen Haken (35B) umfasst.

9. Vorrichtung nach einem der vorherigen Ansprüche, **dadurch gekennzeichnet, dass** die Endoprothese (11) koaxial an dem Stent (13) montiert ist.

10. Vorrichtung nach einem der vorherigen Ansprüche, **dadurch gekennzeichnet, dass** das oder jedes fadenförmige Halteband (33A, 33B) an der Endoprothese (11) gelöst werden kann, wobei der Haltestab (31A, 31B) Mittel (203) zum Halten von wenigstens einem Halteabschnitt (201) des fadenförmigen Haltebandes (33A, 33B) separat von der Schlaufe (45) umfasst, wobei der Abschnitt (201) von den Haltemitteln (203) gehalten wird, wenn die Schlaufe (45) vom Haltehaken (45) gelöst wird, um eine gemeinsame Bewegung des fadenförmigen Haltebandes (33A, 33B) und des Haltestabs (31A, 31B) zuzulassen.

11. Vorrichtung nach Anspruch 10, **dadurch gekennzeichnet, dass** der Halteabschnitt (201) des fadenförmigen Haltebandes (33A, 33B) eine Zusatzschlaufe bildet, die in dem Stent (13) durch eine Halteöffnung (23A, 23B) eingehakt wird, wobei der Haltestab einen zusätzlichen Haltehaken (203) der Zusatzschlaufe (201) hat, der proximal in Bezug auf den Haltehaken (35) der Schlaufe (45) öffnet.

12. Verfahren zur Herstellung einer Vorrichtung nach einem der vorherigen Ansprüche vor deren Implantation in einen Blutkreislaufkanal, **dadurch gekennzeichnet, dass** es die folgenden Schritte beinhaltet:
(a) Halten der Endoprothese (11) in ihrem erweiterten Zustand, wobei die oder jede Schlaufe (45) in einen Haken (35) eines Haltestabes (31A, 31B) der Schlaufe (45) eingreift;
(b) Bewegen des Haltestabes (31A, 31B) von seiner Löseposition in die Halteposition der Schlaufe (45), um zu bewirken, dass die Halteschleife (43) festgehalten und die Endoprothese (11) in ihren eingeschobenen Zustand geführt wird.
